# NEUE EUROPÄISCHE PATENTSCHRIFT

(11) **EP 2 804 734 B2**
(45) Veröffentlichungstag und Bekanntmachung des Hinweises auf die Entscheidung über den Einspruch: **15.01.2025**
(45) Hinweis auf die Patenterteilung: 13.04.2016
(21) Anmeldenummer: 13700903.1
(22) Anmeldetag: 21.01.2013
(51) Int. Cl.: B29C 43/24, A61K 9/70

(54) **GITTERVERLUSTREDUKTION BEI PFLASTERHERSTELLUNG**
REDUCED LOSS OF LATTICE MATERIAL IN PATCH MANUFACTURING
PERTE RÉDUITE DU MATERIAL DE GRILLE PENDANT LA PRODUCTION DES EMPLÂTRES

(30) Priorität: 20.01.2012 EP 12152009
(43) Veröffentlichungstag der Anmeldung: 26.11.2014
(73) Patentinhaber: Luye Pharma AG, 83714 Miesbach (DE)
(72) Erfinder: GRADER, Ludwig, 56626 Andernach (DE); PIOTROWSKI, Holger, 83727 Schliersee (DE)
(74) Vertreter: Kraus & Lederer PartGmbB
(86) Internationale Anmeldenummer: PCT/EP2013/051058
(87) Internationale Veröffentlichungsnummer: WO 2013/107909

(56) Entgegenhaltungen:
- EP-A2- 0 848 937
- WO-A2-00/62763
- DE-A1- 102008 059 054
- DE-A1- 19 946 384
- DE-A1- 3 432 331
- DE-C1- 19 837 764
- US-A- 4 164 170
- US-A- 4 681 001

## Beschreibung

Die vorliegende Erfindung bezieht sich auf Verfahren zur Herstellung von Systemen zur transdermalen oder permukosalen Verabreichung von Wirkstoffen und insbesondere von transdermalen therapeutischen Systemen (TTS), deren Wirkstoffdepots eine von einer rechteckförmigen Gestaltung abweichende Form aufweisen.

Transdermale therapeutische Systeme umfassen gewöhnlich ein Wirkstoffdepot, das auf einer Seite von einer wirkstoffundurchlässigen Rückschicht und auf der dieser gegenüberliegenden Applikationsseite von einer Schutzfolie ganzflächig bedeckt ist. Die häufig mehrteilige Schutzfolie wird vor einer Applikation des Systems entfernt, um dessen Befestigung auf der Haut eines Patienten zu ermöglichen.

Zur Herstellung transdermaler therapeutischer Systeme wird zunächst eine wirkstoffhaltige Beschichtung auf eine Trägerfolienbahn aufgebracht, (vgl. DE 10 2008 059 054 A1). Die Beschichtung weist je nach Art des daraus herzustellenden Wirkstoffdepots einen unterschiedlichen Aufbau auf. Gegenwärtig sind zwei Grundtypen transdermaler therapeutischer Systeme bekannt, Matrixsysteme und Reservoirsysteme. Bei den sogenannten Matrixsystemen ist der Wirkstoff in einer Polymermatrix enthalten, die zumeist aus einem selbsthaftenden druckempfindlichen Polymer ("pressure sensitive adhesive", PSA) gebildet ist. Die Wirkstoffabgabe wird bei diesen Systemen ausschließlich über das Konzentrationsgefälle zur Haut gesteuert. Bei den sogenannten Reservoirsystemen ist der Wirkstoff in einem flüssigen, halbfesten oder festen Reservoir enthalten, wobei zur Regulierung der Wirkstoffabgabe üblicherweise eine Membran verwendet wird, die sich in der Regel an der zur Schutzfolie weisenden Applikationsseite des Wirkstoffdepots befindet.

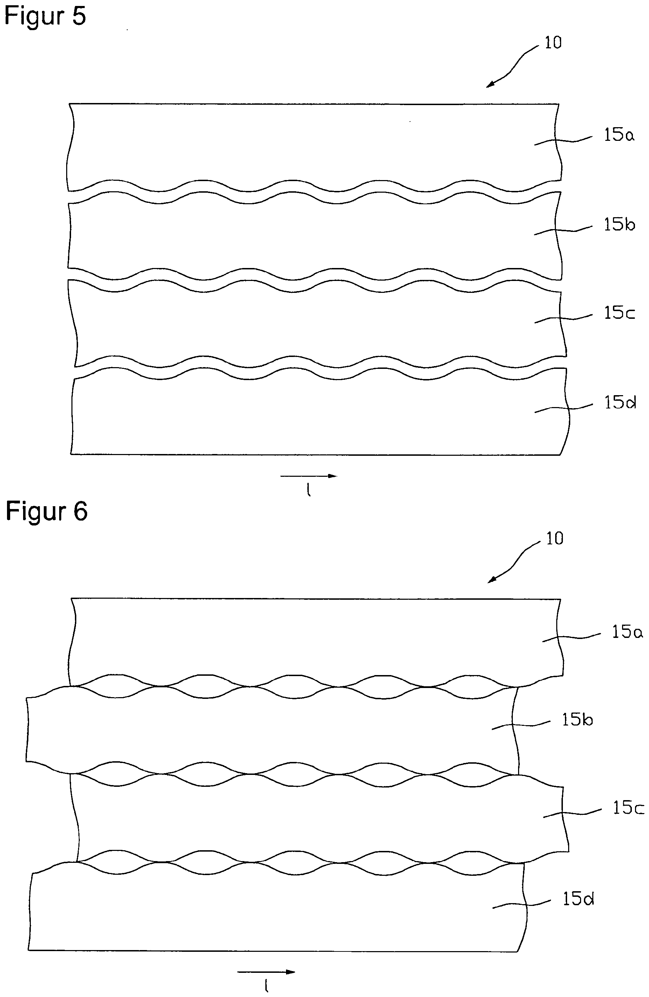

WO0062763 zeigt transdermale Systeme, die eine Trägerfolienbahn und eine haftklebende wirkstoffhaltige Beschichtung aufweisen und die über kreisrunde Wirkstoffdepotflächen verfügen - aus der Definition von Nr. 155 geht hervor, dass es sich sogar um Vertiefungen für die Aufnahme von mehr Wirkstoff handeln kann (siehe Figur 5g). So wie die Aufteilung der Depotzonen in Abbildung 5a-5f getroffen wurde, scheint es, dass der Anteil der Oberfläche der beschichteten Trägerfolienbahn, der nicht als Wirkstoffdepotfläche genutzt ist, weniger als 39 der Gesamtfläche der Trägerfolienbahn beträgt. Nach Durchtrennung der Teilbahnen liegt eine Reihe von Depots hintereinander in einer Reihe.

DE 102008059054 legt im Beispiel 1 eine Mischung aus Polyol mit 25 mg/g Scopolamin und Polyisocyanat offen, wobei diese Mischung in kreisrunde Depots gefüllt wurde. Die kreisrunden Transdermalpflaster wurden anschließend mit einer Fläche von 5 cm² ausgestanzt.

Im weiteren Verlauf des Herstellungsprozesses werden die zur Ausbildung der einzelnen Wirkstoffdepots verwendeten Flächen in der ein- oder mehrlagigen wirkstoffhaltigen Beschichtung vereinzelt, wozu meist ein kontinuierliches oder diskontinuierliches Konturstanzverfahren eingesetzt wird. Die Bahnbreite der zur Herstellung verwendeten Trägerfolie übersteigt die Abmessungen der einzelnen, pro Wirkstoffdepot notwendigen Fläche im Allgemeinen um ein Mehrfaches, so dass zur besseren Nutzung der wirkstoffhaltigen Beschichtung mehrere Wirkstoffdepotflächen in Querrichtung der Trägerfolienbahn nebeneinander angeordnet werden. Bei Wirkstoffdepots mit rechteckförmiger Grundfläche kann so abgesehen von eventuellen Randzonen der Beschichtungslage eine nahezu hundertprozentige Nutzung der wirkstoffhaltigen Beschichtung erzielt werden.

Weisen die zu vereinzelnden Wirkstoffdepots oder die für den Prozessablauf pro Wirkstoffdepot notwendige Fläche jedoch keine rechteckförmigen Grundrisse auf, so können die Umrisslinien nicht aneinander anschließend angeordnet werden, wodurch für das spätere Produkt nicht als Wirkstoffdepot nutzbare Bereiche entstehen. Werden beispielsweise Wirkstoffdepots mit kreisförmigen Grundflächen nebeneinander so angeordnet, dass sich ihre Außenkannten berühren, dann beträgt der Verlust, d. h. der nicht als Wirkstoffdepot genutzte Anteil der wirkstoffhaltigen Beschichtung, unter Nichtberücksichtigung von Randzonen knapp 22 Prozent.

Eine derartige idealisierte Anordnung der Wirkstoffdepots ist in einem technischen Herstellungsprozess praktisch jedoch nicht möglich, da im weiteren Verlauf des Verfahrens die Wirkstoffdepotflächen von den nicht als Wirkstoffdepots genutzten Flächen getrennt werden müssen. Berühren sich die Außenkanten der Wirkstoffdepots, so entstehen zwischen den Wirkstoffdepotflächen einzelne, voneinander isolierte, nicht genutzte Flächen, die einzeln von den Wirkstoffdepots getrennt werden müssten. Ein derartiges Verfahren wäre fehleranfällig und unökonomisch, so dass die nicht für Wirkstoffdepots genutzte Fläche üblicherweise an einem Stück, vorzugsweise durch Abziehen entfernt wird (sogenanntes Abgittern). Hierzu ist es notwendig, dass die abzuziehende Gitterstruktur an keiner Stelle eine Mindestbreite unterschreitet, damit das Gitter nicht reißt.

Der nichtgenutzte Teil der wirkstoffhaltigen Beschichtung bildet so einen Gitter genannten zusammenhängenden Bereich und kann daher im weiteren Verfahren leicht von der Trägerfolienbahn abgezogen werden. Werden beispielsweise runde Wirkstoffdepots mit Durchmessern von 37,5 mm so matrixförmig nebeneinander angeordnet, dass der auch als Gitterstegbreite bezeichnete kleinste Abstand zwischen jeweils zwei zueinander benachbarten Wirkstoffdepots 5 mm beträgt, dann erhält man einen Gitterverlust, d. h. einen Anteil des nicht von den Wirkstoffdepots genutzten Bereichs der wirkstoffhaltigen Beschichtung, von bereits 39 %, der bei sehr teuren Wirkstoffen in äußerst gravierenden Mehrkosten ausdrücken kann.

Es ist daher wünschenswert die Herstellung transdermaler therapeutischer Systeme so zu gestalten, dass die Nutzung der wirkstoffhaltigen Beschichtung bei nicht rechteckförmigen Flächengeometrien für ein Wirkstoffdepot verbessert wird.

Die vorliegende Erfindung basiert nun darauf, dass entsprechende Wirkstoffdepotflächen auf einer mit dem Wirkstoff beschichteten Trägerfolienbahn in parallelen Reihen so angeordnet werden, dass der Anteil der Oberfläche der beschichteten Trägerfolienbahn, der nicht als Wirkstoffdepotfläche genutzt wird, unter Nichtberücksichtigung der Randzonen der beschichteten Trägerfolienbahn weniger als 39 % der Gesamtfläche der beschichteten Trägerfolienbahn beträgt. Randzonen sind dabei als diejenigen Bereiche der beschichteten Trägerfolienbahn definiert, die sich in Bahnrichtung außerhalb, d.h. zu den Rändern hin, einer Linie befinden, die als Tangente an die Wirkstoffdepotflächen anliegt, die die jeweils äußere Reihe der Wirkstoffdepotflächen bildet. Diese beiden Randzonen der beschichteten Trägerfolienbahn werden bei der Berechnung der Gesamtfläche der beschichteten Trägerfolienbahn also nicht berücksichtigt.

In dieser ersten Ausführungsform betrifft die vorliegende Erfindung somit ein Verfahren zur Herstellung von Systemen zur transdermalen oder permukosalen Verabreichung von Wirkstoffen, wobei das Verfahren folgende Schritte umfasst wie in Anspruch 1 dargestellt:
- Bereitstellen einer beschichteten Trägerfolienbahn (10), umfassend eine Trägerfolienbahn (1) mit einer darauf haftklebenden wirkstoffhaltigen Beschichtung (2), wobei auf der beschichteten Trägerfolienbahn (10) Wirkstoffdepotflächen (12) derart definiert sind, dass die Wirkstoffdepotflächen (12) in Bahnrichtung (l) der beschichteten Trägerfolienbahn (10) in zwei oder mehr Reihen derart angeordnet sind, dass sich die Reihen in Bahnrichtung (l) nicht mittels einer geraden Linie voneinander trennen lassen, ohne dabei Wirkstoffdepotflächen (12) zu schneiden, und dass der Anteil der Oberfläche der beschichteten Trägerfolienbahn (10), der nicht als Wirkstoffdepotfläche (12) genutzt wird, unter Nichtberücksichtigung der Randzonen der beschichteten Trägerfolienbahn (10), weniger als 39 % der Gesamtfläche der beschichteten Trägerfolienbahn (10) beträgt; und
- Durchtrennen (S4) der beschichteten Trägerfolienbahn (10) in Bahnrichtung (l) in zwei oder mehr Teilbahnen (15a, 15b, 15c, 15d) derart, dass jede Teilbahn eine Reihe von Wirkstoffdepotflächen (12) enthält, wobei beim Durchtrennen keine der Wirkstoffdepotflächen (12) geschnitten wird.

In diesem Verfahren sind die zwei oder mehr Reihen der Wirkstoffdepotflächen auf der beschichteten Trägerfolienbahn vorzugsweise in parallelen Reihen angeordnet.

Um das vorstehend beschriebene Problem beim Entfernen des nicht zu den Wirkstoffdepots gehörenden Teils der Beschichtung (Stanzgitterabfall) durch Abgittern zu lösen, schlägt die vorliegende Erfindung in einer anderen Ausführungsform vor, die Wirkstoffdepotflächen derart zu definieren, dass sich die Reihen in Bahnrichtung nicht mittels einer geraden Linie voneinander trennen lassen, ohne dass diese Linie eine Abstand d/2 von den Wirkstoffdepotflächen unterschreitet, wobei d als der für das Abgittern notwendige Mindestabstand zwischen zwei Wirkstoffdepotflächen definiert ist.

In dieser zweiten Ausführungsform betrifft die vorliegende Erfindung ein Verfahren zur Herstellung von Systemen zur transdermalen oder permukosalen Verabreichung von Wirkstoffen, wobei das Verfahren folgende Schritte umfasst:
- Bereitstellen einer beschichteten Trägerfolienbahn (10), umfassend einer Trägerfolienbahn (1) mit einer darauf haftklebenden wirkstoffhaltigen Beschichtung (2) wobei auf der beschichteten Trägerfolienbahn (10) Wirkstoffdepotflächen (12) derart definiert sind, dass die Wirkstoffdepotflächen (12) in Bahnrichtung (l) der beschichteten Trägerfolienbahn (10) in zwei oder mehr (vorzugsweise parallelen) Reihen derart angeordnet sind, dass sich die Reihen in Bahnrichtung (l) nicht mittels einer geraden Linie voneinander trennen lassen, ohne dass diese Linie einen Abstand d/2 von den Wirkstoffdepotflächen (12) unterschreitet, wobei d als der für das Abgittern notwendige Mindestabstand zwischenzwei Wirkstoffdepotflächen (12) definiert ist; und
- Durchtrennen (S4) der beschichteten Trägerfolienbahn (10) in Bahnrichtung (l) in zwei oder mehr Teilbahnen (15a, 15b, 15c, 15d) derart, dass jede Teilbahn eine Reihe von Wirkstoffdepotflächen (12) enthält.

Der für das Abgittern notwendige Mindestabstand d der Wirkstoffdepots voneinander hängt von verschiedenen Faktoren ab und kann vom Fachmann für den jeweiligen Einzelfall leicht bestimmt werden. Die notwendige Breite hängt insbesondere vom Material der wirkstoffhaltigen Beschichtung ab. Je reißfester dieses Material ist, desto kleiner kann dir Breite der in dem Stanzgitter verbleibenden "Brücken" sein, ohne dass es beim Abgittern, d.h. Abziehen des Stanzgitters, zu einem Reißen des Gitters kommt. Andere Faktoren sind die Stärke des Anhaftens der wirkstoffhaltigen Beschichtung an der Trägerfolienbahn sowie die Verarbeitungsgeschwindigkeit und die Ausgestaltung der Abgittervorrichtung. Außerdem hat die Größe der Wirkstoffdepotflächen einen Einfluss auf die Stabilität des Stanzgitterabfalls. Letztendlich können all diese Faktoren sowie die daraus resultierende Mindestbreite d der Gitterbrücken beispielsweise durch einfach praktische Versuche vom Fachmann bestimmt werden.

In der Praxis haben sich beispielsweise Mindestabstände d zwischen zwei Wirkstoffdepotflächen von 10 mm, 9 mm, 8 mm, 7 mm, 6 mm, 5 mm, 4 mm, 3 mm, 2 mm oder 1 mm bewährt.

In einer Ausführungsform basiert die vorliegende Erfindung darauf, dass entsprechende Wirkstoffdepotflächen auf einer mit dem Wirkstoff beschichteten Trägerfolienbahn in parallelen Reihen so angeordnet werden, dass sich benachbarte Reihen derart überlappen, dass nicht genutzte Flächen einer Reihe von Wirkstoffdepotflächen einer benachbarten Reihe zumindest teilweise eingenommen werden. Dies führt jedoch wiederum zu dem Problem, dass entsprechende Trägerfolienbahnen nicht wie sonst üblich in gerade, streifenförmige Teilbahnen geschnitten werden können, da hierdurch Wirkstoffdepotflächen geschnitten würden und die somit erhaltenen Wirkstoffdepots unbrauchbar würden. Um dieses Problem zu lösen, wird das erfindungsgemäße Verfahren gemäß Anspruch 1 zur Verfügung gestellt.

Das erfindungsgemäße Verfahren hat den Vorteil, dass das Durchtrennen der beschichteten Trägerfolienbahn in Bahnrichtung in zwei oder mehr Teilbahnen nicht geradlinig erfolgt, sondern beispielsweise wellenförmig derart, dass beim Durchtrennen der beschichteten Trägerfolienbahn keine der Wirkstoffdepotflächen geschnitten wird. Dies ermöglicht ein optimales Ausnutzen der Fläche der beschichteten Trägerfolienbahn ohne das Durchschneiden von Wirkstoffdepotflächen beim Auftrennen der Trägerfolienbahn in einzelne Teilbahnen, so dass alle erhaltenen Wirkstoffdepotflächen tatsächlich als Wirkstoffdepots genutzt werden können.

Die so erhaltenen Teilbahnen, die die Wirkstoffdepotflächen in einer Reihe angeordnet enthalten, können dann entweder getrennt in Einzelbereiche geschnitten werden, die jeweils nur ein Wirkstoffdepot enthalten oder die Wirkstoffdepots können einzeln von den Teilbahnen abgelöst und weiterverarbeitet werden.

Vorteilhaft ist es jedoch, wenn die aus einer beschichteten Trägerfolienbahn erhaltenen Teilbahnen parallel weiterverarbeitet werden können. Hierzu weisen die nach dem vorstehend beschriebenen erfindungsgemäßen Verfahren erhaltenen Teilbahnen zunächst jedoch noch den Nachteil auf, dass die einzelnen Wirkstoffdepotflächen paralleler Teilbahnen in Bahnrichtung versetzt vorliegen. Mit anderen Worten, befinden sich die Wirkstoffdepots auf benachbarten Teilbahnen nicht exakt an der gleichen Stelle in Richtung der Bahnrichtung, so dass eine maschinelle Weiterverarbeitung hierdurch erschwert wird. Dieses zusätzliche Problem wird durch die in Anspruch 2 beschriebenen bevorzugten Ausführungsformen des erfindungsgemäßen Verfahrens gelöst.

Hiernach kann das erfindungsgemäße Verfahren als weiteren Schritt das Verändern der Lage der Teilbahnen relativ zueinander umfassen, so dass keine der Teilbahnen seitlich in eine der anderen Teilbahnen eingreift. Die Teilbahnen werden durch diesen Verfahrensschritt voneinander beabstandet, so dass sie leichter weiter bearbeitet werden können und insbesondere ein Verschieben der Teilbahnen in Bahnrichtung relativ zueinander ermöglicht wird.

In einer weiteren bevorzugten Ausführungsform umfasst das erfindungsgemäße Verfahren als zusätzlichen Schritt das Verschieben der Teilbahnen relativ zueinander in Bahnrichtung so, dass die parallel liegenden Teilbahnen in einer Richtung 90° zur Bahnrichtung in geraden Linien so durchtrennt werden können, dass Einzelabschnitte erhalten werden, die jeweils nur eine Wirkstoffdepotfläche enthalten, und wobei beim Durchtrennen keine der Wirkstoffdepotflächen geschnitten wird. Das Verschieben der Teilbahnen ermöglicht es, anschließend die einzelnen, sich auf parallelen Teilbahnen nebeneinander befindlichen Wirkstoffdepots parallel in einer geraden Linie weiter zu verarbeiten. Dies erleichtert das weitere Verarbeiten beispielsweise in einer kontinuierlichen Bearbeitungsvorrichtung.

In dem erfindungsgemäßen Verfahren handelt es sich bei der Bahnrichtung in der Regel um die Richtung der längeren Seitenkante der beschichteten Trägerfolienbahn. In der Regel weisen entsprechende Trägerfolienbahnen eine relativ geringe Breite und eine Länge auf, die deren Breite um ein Vielfaches übersteigt. Die Bahnrichtung verläuft dann in Richtung der Länge der Trägerfolienbahn.

In dem erfindungsgemäßen Verfahren ist es wichtig, dass die Wirkstoffdepotflächen weder in Bahnrichtung der beschichteten Trägerfolienbahn noch quer dazu geschnitten werden. Dies bedeutet, dass beim Durchtrennen der beschichteten Trägerfolienbahn oder auch nur der wirkstoffhaltigen Beschichtung keine der Wirkstoffdepotflächen durchtrennt wird. Mit anderen Worten dürfen Trennlinien maximal tangential an einer Wirkstoffdepotfläche entlang laufen, nicht jedoch durch eine solche Fläche.

In einer weiteren Ausführungsform des erfindungsgemäßen Verfahrens sind die Wirkstoffdepotflächen so angeordnet, dass die Anordnung keine vierzählige Drehachse aufweist, vorzugsweise so, dass die Anordnung die Symmetrieelemente der Gruppe p6m aufweist.

In einer weiteren, vorteilhaften Ausführungsform ist das erfindungsgemäße Verfahren so ausgestaltet, dass der Anteil der Oberfläche der beschichteten Trägerfolienbahn, der nicht als Wirkstoffdepotfläche genutzt wird, unter Nichtberücksichtigung der Randzonen weniger als 38 %, 37 %, 36 %, 35 %, 34 %, 33 %, 32 %, 31 %, 30 %, 29 %, 28 %, 27 %, 26 %, 25 %, 24 %, 23 %, 22 % oder sogar 21 % der Gesamtfläche der beschichteten Trägerfolienbahn beträgt.

Die Wirkstoffdepotflächen können durch Formstanzen der beschichteten Trägerfolien definiert werden. So ist es beispielsweise möglich, dass in eine beschichtete Trägerfolienbahn die Wirkstoffdepotflächen zunächst durch Formstanzen definiert werden und die anderen Verfahrensschritte, also das Durchtrennen der Trägerfolienbahn in Teilbahnen, etc., anschließend durchgeführt werden. Es ist aber auch möglich, dass die Wirkstoffdepotflächen zunächst nur abstrakt auf der beschichteten Trägerfolienbahn definiert werden und das tatsächliche Formen der Wirkstoffdepots beispielsweise durch Formstanzen der Wirkstoffdepotflächen erst nach Durchtrennen der beschichteten Trägerfolienbahn in zwei oder mehr Teilbahnen oder zu einem noch späteren Zeitpunkt nach Durchführung weiterer anderer Verfahrensschritte erfolgt.

Ebenfalls zu einem beliebigen Zeitpunkt innerhalb des erfindungsgemäßen Verfahrens, vorzugsweise jedoch nach Erhalt der Wirkstoffdepots beispielsweise durch Formstanzen und vor dem Durchtrennen der Trägerfolienbahn in Teilbahnen kann der nicht als Wirkstoffdepotfläche definierte Bereich der beschichteten Trägerfolienbahn entfernt werden, beispielsweise durch sogenanntes Abgittern.

In einer Ausführungsform der vorliegenden Erfindung, die wiederum abhängig oder unabhängig von dem Verfahren gemäß der oben beschriebenen ersten Ausführungsform sein kann, wird das Verfahren derart ausgeführt, dass anstelle der Definition der Wirkstoffdepotflächen auf der beschichteten Trägerfolienbahn eine Anordnung sich nicht überlappender Zellbereiche auf der wirkstoffhaltigen Beschichtung bestimmt wird, wobei die Zellbereiche die maximale Ausdehnung der Wirkstoffdepotflächen definieren, die Wirkstoffdepotflächen aber auch kleiner sein können, so dass sie nur einen Teil der Zellbereiche einnehmen. Bei dieser Definition der Erfindung wird das Verfahren dadurch gekennzeichnet, dass die Anordnung der sich nicht überlappenden Zellbereiche so gewählt wird, dass der Schritt des Verschiebens der einzelnen Teilbahnen relativ zueinander in Bahnrichtung ohne den vorangehenden Schritt der Veränderung der Lage der Teilbahnen relativ zueinander so, dass keine der Teilbahnen seitlich in eine andere Teilbahn eingreift, nicht ohne Überlappungen benachbarter Teilbahnen möglich ist.

Ausführungsformen einer solchen Herstellung umfassen ein Verfahren, das Schritte umfasst zum Bereitstellen einer Trägerfolienbahn mit einer darauf haftklebenden wirkstoffhaltigen Beschichtung, zum Bestimmen einer Anordnung sich nicht überlappender Zellbereiche auf der wirkstoffhaltigen Beschichtung, zum Durchtrennen der beschichteten Trägerfolienbahn in zwei oder mehr Teilbahnen so, dass jede der Trennlinien ausschließlich unmittelbar quer zur Bahnrichtung der Trägerfolie zueinander benachbart angeordnete Zellbereiche voneinander trennt, zum Verändern (S5) der Lage der Teilbahnen relativ zueinander so, dass keine der Teilbahnen seitlich in eine der anderen Teilbahnen eingreift, und zum Verschieben (S6) der einzelnen Teilbahnen relativ zueinander in Bahnrichtung der Trägerfolie so, dass quer zur Bahnrichtung nächst benachbarte Zellbereiche zueinander in Bahnrichtung keinen Versatz aufweisen, wobei die Anordnung der sich nicht überlappenden Zellbereiche so gewählt wird, dass Schritt (S6) ohne vorangehenden Schritt (S5) nicht ohne Überlappungen benachbarter Teilbahnen möglich ist.

In einer bevorzugten Ausführungsform dieses Verfahrens wird die Anordnung der sich nicht überlappenden Zellbereiche auf der wirkstoffhaltigen Beschichtung so bestimmt, dass die Summe der einzelnen Querausdehnungen zweier in Querrichtung der Trägerfolie nächst benachbart angeordneter Zellbereiche größer als die gesamte Querausdehnung der beiden Zellbereiche ist.

Auch in dieser Ausführungsform des erfindungsgemäßen Verfahrens ist es vorteilhaft, wenn die Anordnung der sich überlappenden Zellbereiche so gewählt wird, dass diese Anordnung keinen vierzähligen Drehpunkt aufweist, insbesondere so, dass in den Zellbereichen kreisrunde Wirkstoffdepots angeordnet werden können, so dass die Anordnung der kreisrunden Wirkstoffdepots die Symmetrieelemente der Gruppe p6m aufweist.

Darüber hinaus ist es auch vorteilhaft, wenn die Anordnung der sich nicht überlappenden Zellbereiche so gewählt wird, dass in den Zellbereichen kreisrunde Wirkstoffdepots so angeordnet werden können, dass der nicht als Wirkstoffdepot genutzte Anteil der wirkstoffhaltigen Beschichtung unter Nichtberücksichtigung von Randzonen weniger als 38 %, 37 %, 36 %, 35 %, 34 %, 33 %, 32 %, 31 %, 30 %, 29 %, 28 %, 27 %, 26 %, 25 %, 24 %, 23 %, 22 % oder sogar 21 % der Gesamtfläche der beschichteten Trägerfolienbahn beträgt, wobei die Randzonen wie vorstehend definiert sind.

Das Verfahren ermöglicht eine ineinandergreifende Anordnung der Zellbereiche in Richtung quer zur Längsausbreitung der noch unbearbeiteten Trägerfolie und hierüber eine verbesserte Nutzung der Beschichtung zur Ausbildung von Wirkstoffdepots für die Verwendung in transdermalen therapeutischen Systemen.

Die Herstellung erfolgt vorteilhaft unter Verwendung einer Vorrichtung, die nachfolgend aufgeführte Einrichtungen umfasst. Die Vorrichtung fällt nicht unter den Schutz der vorliegenden Erfindung und spiegelt lediglich die Offenbarung wider. Eine Einrichtung, die zum Bereitstellen einer Trägerfolienbahn, auf der eine wirkstoffhaltige Beschichtung haftklebend aufgebracht ist, ausgebildet ist. Eine Trenneinrichtung, die so zum Durchtrennen der beschichteten Trägerfolienbahn in zwei oder mehr Teilbahnen ausgebildet ist, dass jede von der Trenneinrichtung in die Trägerfolienbahn eingebrachte Trennlinie ausschließlich unmittelbar quer zur Bahnrichtung der Trägerfolie zueinander benachbart angeordnete Bereiche voneinander trennt, die jeweils zur Ausbildung eines Wirkstoffdepots für ein transdermales therapeutisches System vorgesehen sind. Eine Versetzeinrichtung, die so zum Verändern der Lage der Teilbahnen relativ zueinander ausgebildet ist, dass keine der Teilbahnen seitlich in eine der anderen Teilbahnen eingreift. Und eine Ausgleichseinrichtung, die so zum Verschieben der einzelnen Teilbahnen relativ zueinander in Bahnrichtung der Trägerfolie ausgebildet ist, dass quer zur Bahnrichtung nächst benachbarte, zur Ausbildung von Wirkstoffdepots vorgesehene, Bereiche unterschiedlicher Teilbahnen zueinander in Bahnrichtung keinen Versatz aufweisen.

Um die Teilbahnen vorteilhaft unverschränkt so nebeneinander anordnen zu können, dass keiner der Ränder einer Teilbahn in den Rand einer anderen Teilbahn seitlich eingreift, umfasst bei wie bezeichneten Verfahren das Verändern der Lage der Teilbahnen relativ zueinander vorzugsweise ein Vergrößern des Abstands zwischen den Teilbahnen so, dass die Querausdehnung zweier auf unmittelbar benachbarten Teilbahnen nächst benachbart angeordneter Zellbereiche gleich oder größer als die Summe der einzelnen Querausdehnungen dieser Zellbereiche ist. Zur Durchführung eines solchen Verfahrensschrittes weist die Herstellungsvorrichtung vorzugsweise eine entsprechend ausgebildete Versetzeinrichtung auf. Der seitliche Versatz der Teilbahnen wird bei bevorzugten Ausführungsformen solcher Versetzeinrichtungen mithilfe im Stand der Technik bekannten Schwenkrahmen bewirkt.

Bevorzugte Ausführungsformen des Verfahrens weisen ferner Schritte auf zum Durchtrennen der wirkstoffhaltigen Beschichtung entlang von innerhalb der Zellbereiche angeordneten, in sich geschlossenen linienförmigen Geometrien (=Wirkstoffdepots), wobei der Mindestabstand der Geometrien zu einer Randlinie der Zellbereiche einem vorgegebenen Wert von vorzugsweise kleiner 5 mm entspricht, und zum Entfernen der nicht von den linienförmigen Geometrien umschlossenen Anteile der Zellbereiche. Eine entsprechende, z. B. mittels Form- bzw. Konturstanzen vorgenommene Vereinzelung von Wirkstoffdepots in der Beschichtung ermöglicht die Ausbildung der nicht als Wirkstoffdepot genutzten Anteile der Beschichtung als zusammenhängendes und damit leicht abzuziehendes Gitter.

Entsprechend weisen vorteilhafte Ausführungsformen der Herstellungsvorrichtung der Offenbarung ferner eine Konturierungseinrichtung auf, die zum Durchtrennen der wirkstoffhaltigen Beschichtung entlang von in sich geschlossenen linienförmigen Geometrien ausgebildet ist, wobei die Konturierungseinrichtung ferner so zur Platzierung der Geometrien innerhalb von für die Ausbildung von Wirkstoffdepots vorgesehenen Bereichen ausgebildet, dass der Mindestabstand der Geometrien zu einer Randbegrenzung der Bereiche einem vorgegebenen Wert entspricht, der beispielsweise kleiner 10 mm, kleiner 9 mm, kleiner 8 mm, kleiner 7 mm, kleiner 6 mm, kleiner 5 mm, kleiner 4 mm, kleiner 3 mm, kleiner 2 mm oder kleiner 1 mm sein kann. Weitere bevorzugte Ausführungsformen der Vorrichtung weisen entsprechend eine Abgittereinrichtung auf, die zum Entfernen der nicht von den linienförmigen Geometrien umschlossenen Anteilen der für die Ausbildung von Wirkstoffdepots vorgesehenen Bereiche ausgebildet ist.

Weitere bevorzugte Ausführungsformen des Verfahrens sehen eine Vornahme des Durchtrennens der beschichteten Trägerfolienbahn in Teilbahnen so vor, dass hierdurch die Lage und Ausdehnung der Zellbereiche auf der wirkstoffhaltigen Beschichtung festgelegt, und die Aufteilung bzw. Untergliederung der Beschichtung in Zellbereiche somit vorteilhaft implizit vorgenommen ist.

Bei weiter vorteilhaften Ausführungsformen erfolgt das Verändern der Lage der Teilbahnen relativ zueinander simultan zum Verschieben der einzelnen Teilbahnen relativ zueinander in Bahnrichtung, beispielsweise durch Schrägführen der Teilbahnen auf unterschiedlich langen Wegstrecken.

Das Entfernen der nicht von linienförmigen Geometrien umschlossenen Anteile der Zellbereiche erfolgt bei vorteilhaften Ausführungsformen nach dem Durchtrennen der wirkstoffhaltigen Beschichtung entlang der innerhalb der Zellbereiche angeordneten linienförmigen Geometrien, und dieses nach dem Verändern der Lage der Teilbahnen relativ zueinander und dem Verschieben dieser relativ zueinander in Bahnrichtung.

Bei anderen bevorzugten Ausführungsformen erfolgt das Durchtrennen der beschichteten Trägerfolienbahn in Teilbahnen nach dem Entfernen der nicht von linienförmigen Geometrien umschlossenen Anteile der Zellbereiche, und dieses nach dem Durchtrennen der wirkstoffhaltigen Beschichtung entlang der innerhalb der Zellbereiche angeordneten linienförmigen Geometrien.

Bei ebenfalls bevorzugten Ausführungsformen erfolgt das Entfernen der nicht von linienförmigen Geometrien umschlossenen Anteile der Zellbereiche nach dem Verändern der Lage der Teilbahnen relativ zueinander und dem Verschieben dieser relativ zueinander in Bahnrichtung, und letzteres nach dem Durchtrennen der wirkstoffhaltigen Beschichtung entlang der linienförmigen Geometrien und dem Durchtrennen der beschichteten Trägerfolienbahn in Teilbahnen.

Die beschriebenen Ausführungsformen gestatten eine effektive Nutzung der wirkstoffhaltigen Beschichtung vor allem bei nicht rechteckförmig ausgebildeten Wirkstoffdepots, so dass die in sich geschlossenen linienförmigen Geometrien bei bevorzugten Ausführungsformen des Verfahrens kreisförmig oder ellipsenförmig ausgebildet sind.

Weitere Merkmale der Erfindung ergeben sich aus der nachfolgenden Beschreibung von Ausführungsbeispielen in Verbindung mit den Ansprüchen sowie den beiliegenden Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können bei erfindungsgemäßen Ausführungsformen die bei den nachstehend erläuterten Ausführungsbeispielen angeführten Merkmale in von den Beispielen abweichender Anzahl und Kombination verwirklicht sein. Bei der nachfolgenden Erläuterung einiger Ausführungsbeispiele der Erfindung wird auf die beiliegenden Figuren Bezug genommen, von denen
- Figur 1: den Aufbau einer beschichteten Trägerfolienbahn zur Herstellung transdermaler therapeutischer Systeme in einer schematischen Darstellung zeigt,
- Figur 2: eine beschichtete Trägerfolienbahn mit darauf in herkömmlicher Weise vereinzelten Wirkstoffdepots für transdermale therapeutische Systeme in einer schematisierten Draufsicht veranschaulicht,
- Figur 3: eine Draufsicht auf eine beschichtete Trägerfolienbahn mit graphischer Hervorhebung einer Anordnung von aneinandergrenzenden Elementzellen in einer schematisierten Darstellung zeigt,
- Figur 4: eine Anordnung von Trennlinien für ein Auftrennen der beschichteten Trägerfolienbahn von Figur 3 in Teilbahnen in einer schematisierten Darstellung zeigt,
- Figur 5: eine Schemadarstellung einer entlang der Trennlinien von Figur 4 aufgetrennte Trägerfolienbahn zeigt, deren so erzeugte Teilbahnen seitwärts beabstandet wurden,
- Figur 6: eine schematische Draufsicht auf eine Trägerfolie mit relativ zueinander um einen Längsversatz zwischen Wirkstoffdepots bzw. Zellbereichen benachbarter Reihen verschobenen Teilbahnen zeigt,
- Figur 7: die Vereinzelung von Wirkstoffdepots auf den wirkstoffhaltigen Beschichtungen der Teilbahnen schematisch veranschaulicht,
- Figur 8: ein Beispiel für eine flächennutzende Vereinzelung von runden Wirkstoffdepots auf einer beschichteten Trägerfolie in einer schematischen Darstellung zeigt,
- Figur 9: die in Teilbahnen aufgetrennte beschichtete Trägerfolie von Figur 8 schematisch illustriert,
- Figur 10: eine schematische Illustration der Teilbahnen von Figur 9 nach dem lateralen Beabstanden, Längsversatzausgleich und Abgittern wiedergibt,
- Figur 11: eine Ausführungsform eines Verfahrens zur besseren Nutzung der wirkstoffhaltigen Beschichtung in Form eines Flussdiagramms veranschaulicht und
- Figur 12: eine Vorrichtung der Offenbarung zur Herstellung transdermaler therapeutischer Systeme unter verbesserter Wirkstoffnutzung in einer funktionsschematisierten Seitenansicht und Draufsicht veranschaulicht.

In den Figuren werden gleiche oder ähnliche Bezugszeichen für funktionell gleichwertige oder ähnliche Charakteristiken unabhängig von speziellen Ausführungsformen verwendet.

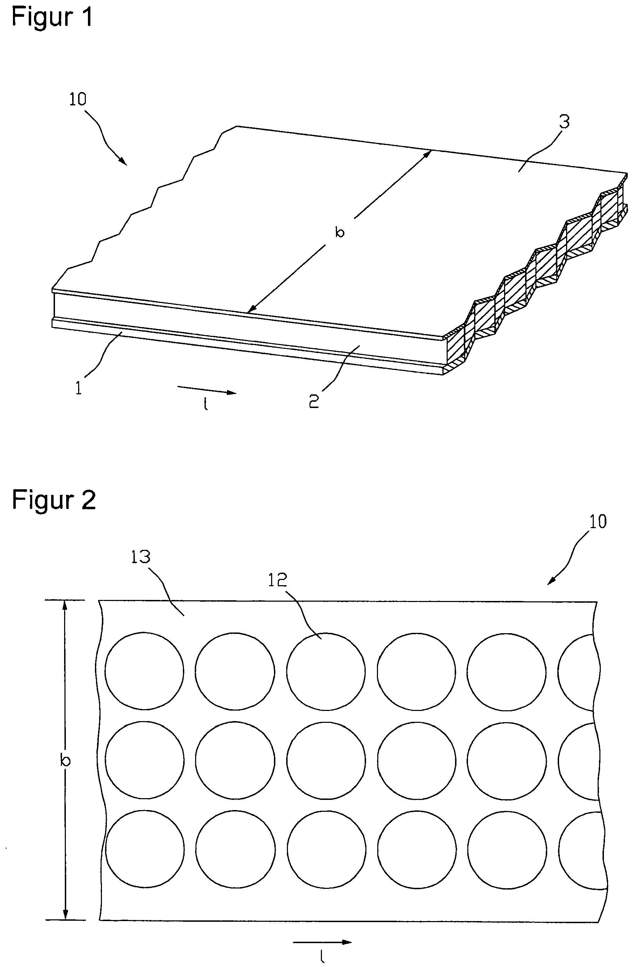

Figur 1 veranschaulicht den Aufbau einer beschichteten Trägerfolienbahn 10 zur Herstellung transdermaler therapeutischer Systeme. Zur besseren Verdeutlichung des Sachverhalts ist die Darstellung nicht maßstabsgetreu ausgeführt. Die beschichtete Trägerfolienbahn 10, von der in Figur 1 nur ein Teilstück gezeigt ist, weist eine bestimmte Breite b und eine im Verhältnis zur Breite wesentlich größere Länge l auf. Die beschichtete Trägerfolienbahn 10 besteht wenigstens aus zwei Schichten, der Trägerfolienbahn 1 und einer darauf aufgebrachten wirkstoffhaltigen Beschichtung 2. Üblicherweise ist die der Trägerfolienbahn 1 abgewandte Seite der wirkstoffhaltigen Beschichtung 2, wie im in Figur 1 veranschaulichten Beispiel gezeigt ist, zusätzlich von einer darauf aufgebrachten Deckfolie 3 abgedeckt. Abhängig vom jeweiligen Anwendungsfall erstreckt sich die wirkstoffhaltige Beschichtung 2 über die gesamte Breite b der Trägerfolienbahn, wie in Figur 1 gezeigt nur über einen Teil deren Breite, oder ist in Form mehrerer nebeneinander angeordneter Einzelbahnen auf der Trägerfolie 1 aufgebracht. Für die Zwecke einer wie nachstehend beschrieben verbesserten Nutzung der wirkstoffhaltigen Beschichtung 2 erstreckt sich diese über die gesamte Breite b der Trägerfolienbahn 1 oder zumindest nahezu über die gesamte Breite b der Trägerfolienbahn 1. Je nach Art der aus der beschichteten Trägerfolienbahn 10 herzustellenden transdermalen therapeutischen Systeme kann die wirkstoffhaltige Beschichtung 2 einlagig oder mehrlagig aufgebaut sein und auch eine Membran umfassen.

Die Breite der beschichteten Trägerfolienbahn 10 richtet sich außer nach dem jeweils herzustellenden transdermalen therapeutischen System in erster Linie nach den Gegebenheiten der jeweils zur Herstellung verwendeten Apparatur. Die verwendeten Bahnbreiten betragen meist ein Mehrfaches der Breiten daraus herzustellender transdermaler therapeutischer Systeme. Um die beschichtete Trägerfolienbahn besser auszunutzen, erfolgt die Vereinzelung von Wirkstoffdepots 12 in der wirkstoffhaltigen Beschichtung 2 daher in der Regel in einer mehrere Reihen umfassenden Anordnung. Eine solche matrixförmige Anordnung von Wirkstoffdepots 12 ist in Figur 2 veranschaulicht. Die einzelnen Wirkstoffdepots 12, von denen in der Figur nur eines stellvertretend für alle mit einem Bezugszeichen versehen ist, sind bei der dargestellten Anordnung in drei Reihen angeordnet. Die sich in Längsrichtung der beschichteten Trägerfolienbahn 10 erstreckenden Reihen sind bezüglich der Breite der Folienbahn 10 nebeneinander angeordnet. Die zueinander benachbarten Wirkstoffdepots unterschiedlicher Reihen weisen keinen Versatz zueinander in Längsrichtung der Trägerfolienbahn 10 auf, sondern befinden sich im Wesentlichen jeweils an derselben Längenposition der beschichteten Trägerfolienbahn 10. Mit anderen Worten entspricht die Anordnung der Wirkstoffdepots 12 einer nach Reihen und Spalten gegliederten Feldanordnung, die eine Voraussetzung zum chargenweisen Übertragen aller an der jeweils selben Längenposition der Folienbahn 10 befindlichen Wirkstoffdepots 12 an eine Schutzfolie bzw. zum chargenweisen Trennen der Folienbahn in einzelne Abschnitte im Rahmen eines Abtrennens der einzelnen transdermalen therapeutischen Systeme ist. Unter einer chargenweisen Behandlung der Wirkstoffdepots ist hierbei eine gleichzeitige Behandlung von in einer Spalte der Feldanordnung nebeneinander liegenden Wirkstoffdepots zu verstehen.

Die Wirkstoffdepots 12 sind zueinander sowohl in Längsrichtung der beschichteten Trägerfolienbahn 10 als auch quer hierzu beabstandet, so dass man ein zusammenhängendes Gitter 13 erhält, das von der Trägerfolie leicht abgezogen werden kann.

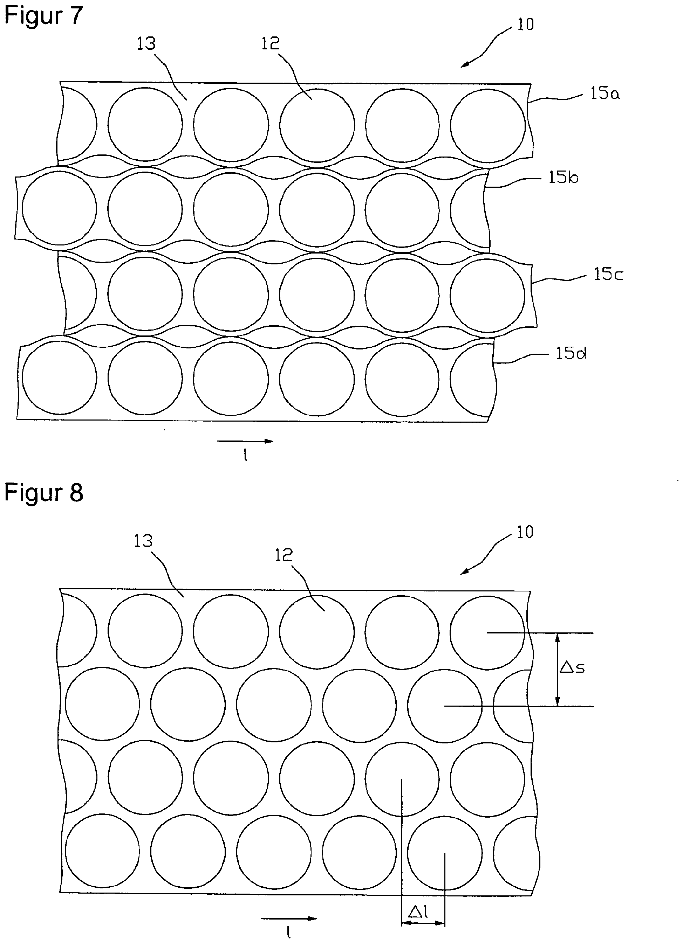

Die zuvor beschriebene nach Reihen und Spalten gegliederte matrixförmige Anordnung der Wirkstoffdepots 12 führt bei nicht rechteckförmigen Wirkstoffdepotgeometrien zu einer geringen Nutzung der wirkstoffhaltigen Beschichtung 2. Eine Anordnung mit verbesserter Nutzung der wirkstoffhaltigen Beschichtung 2 ist in Figur 8 dargestellt. Hier sind die runden Wirkstoffdepots 12 einander benachbarter Reihen bezüglich der Längsrichtung l der beschichteten Trägerfolienbahn 10 versetzt zueinander angeordnet, wobei die Reihen so ineinander eingreifen, dass der zwischen zwei benachbarten Wirkstoffdepots 12 einer Reihe verfügbare Raum von einem Wirkstoffdepot 12 einer zu dieser Reihe unmittelbar benachbart angeordneten Reihe mitgenutzt wird. Mit anderen Worten ist der im Folgenden als Reihenabstand Δs bezeichnete Abstand von quer zur Längsausdehnung der Trägerfolie 10 benachbart angeordneten Wirkstoffdepots 12 bei gleichgroßer Gitterstegbreite kleiner als bei einer Anordnung nach Figur 2.

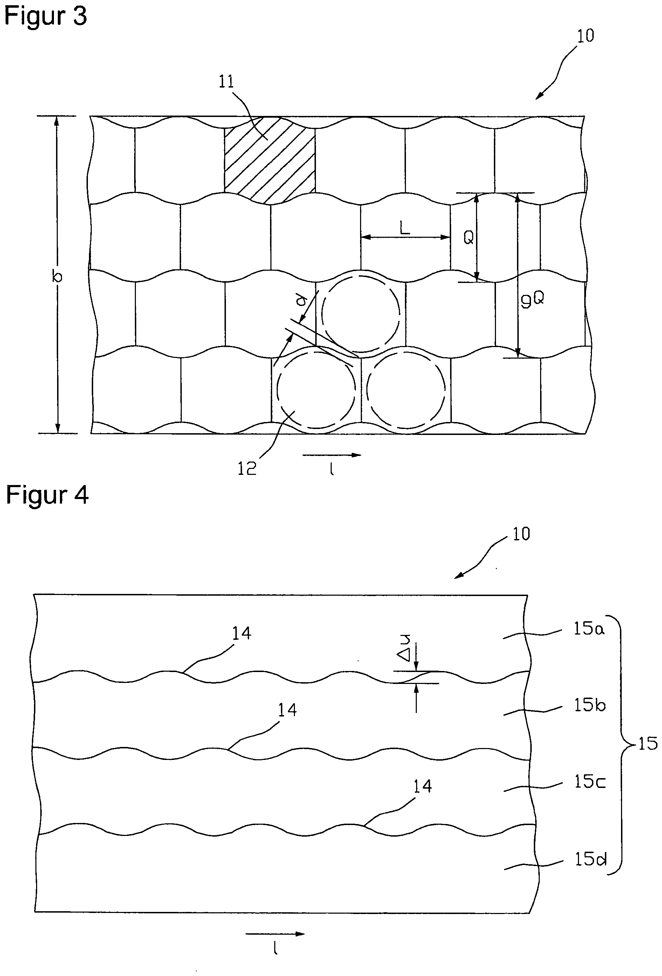

Der Längsversatz Δl zwischen Wirkstoffdepots 12 benachbarter Reihen behindert jedoch eine wie oben beschriebene spaltenförmige chargenweise Weiterbearbeitung. Damit die Wirkstoffdepots 12 der einzelnen Reihen zur Weiterverarbeitung gleichauf, d.h. ohne Längsversatz, angeordnet sind, wird die beschichtete Trägerfolienbahn 10 in mehrere Teilbahnen aufgetrennt, wobei jede der Teilbahnen jeweils eine Reihe von für Wirkstoffdepots 12 vorgesehenen Flächen enthält. Anschließend wird die relative Lage der Teilbahnen zueinander so verändert, dass die für Wirkstoffdepots 12 vorgesehenen Flächen unterschiedlicher Teilbahnen in Querrichtung nebeneinander ohne Längsversatz Δl und ohne Eingriff Δu (siehe Figur 4) angeordnet sind.

Ein erstes Verfahren zur besseren Nutzung der wirkstoffhaltigen Beschichtung 2 durch Wirkstoffdepots 12 wird im Folgenden unter Bezugnahme auf die Figuren 3 bis 7 erläutert. In einem ersten Schritt des Verfahrens wird die wirkstoffhaltige Beschichtung 2 der Breite b in mehrere, vorzugsweise kongruente Zellbereiche 11 untergliedert. Einer der Zellbereiche ist in Figur 3 stellvertretend für alle mit einem Bezugszeichen versehen und zur besseren Erkennung seiner Geometrie schraffiert dargestellt. Die einzelnen Zellbereiche 11 grenzen ohne Zwischenbereiche unmittelbar aneinander an und überlappen sich nicht.

Größe und Form eines Zellbereichs 11 richten sich nach Größe und Form der jeweils herzustellenden Wirkstoffdepots 12 und den für ein Abziehen des Stanzgitters oder für einen Überstand des späteren Teils des Releaseliners, der den Träger der einzelnen Wirkstoffdepots darstellt.. Die in Figur 3 gezeigten Zellbereiche 11 sind für kreisförmige Wirkstoffdepots 12 ausgelegt, deren vorgesehene Lage in drei der Zellbereiche 11 durch gestrichelte Linien angedeutet ist. Die Bestimmung von Form und Anordnung der Zellbereiche erfolgt vorzugsweise im Hinblick auf eine unter gegebenen Bedingungen möglichst dichte Anordnung der zur Ausbildung der Wirkstoffdepots auf der wirkstoffhaltigen Beschichtung 2 vorgesehenen Flächen. Bei dem in Figur 3 gezeigten Beispiel ist die Zellbereichsform auf eine optimale Nutzung der wirkstoffhaltigen Beschichtung 2 unter Wahrung eines Mindestabstands d (Gitterstegbreite) zwischen jeweils unmittelbar zueinander benachbarten Wirkstoffbereichen 12 entwickelt worden.

Bei anderen Formen herzustellender Wirkstoffdepots 12 ergeben sich natürlich Zellbereiche mit Umrandungsgeometrien, die von der in Figur 3 gezeigten abweichen. Außerdem kann die wirkstoffhaltige Beschichtung 2, falls in einem Herstellungsprozess unterschiedlich große oder nicht ähnlich geformte Wirkstoffdepots 12 herzustellen sind, in eine Anordnung unterschiedlich großer und auch unterschiedlich geformter Zellbereiche 11 untergliedert werden.

Bei nicht reckeckförmigen Wirkstoffdepotgeometrien ist eine optimale Nutzung der wirkstoffhaltigen Beschichtung 2 immer dann gegeben, wenn eine Wirkstoffdepotfläche 12 einer Reihe nahe an den Raum zwischen zwei Wirkstoffdepotflächen 12 einer nächst benachbarten Reihe angrenzend bzw. in diesen Raum eingreifend angeordnet ist. Entsprechend kann eine raumnutzende Form und Anordnung von Zellbereichen 11 so charakterisiert werden, dass, wie in Figur 3 veranschaulicht ist, die Summe der einzelnen Querausdehnungen Q zweier in Querrichtung der Trägerfolie 10 nächst benachbart angeordneter Zellbereiche 11 größer ist, als die gesamte Querausdehnung gQ dieser beiden Zellbereiche.

Die beschriebene Bestimmung einer Anordnung sich nicht überlappender Zellbereiche 11 auf der wirkstoffhaltigen Beschichtung 2 ist rein organisatorischer Natur und schlägt sich üblicherweise nicht in einer tatsächlichen Markierung oder Strukturierung der wirkstoffhaltigen Beschichtung 2 nieder. Sie ist jedoch Grundlage für, bzw. ergibt sich indirekt aus der Anordnung der Trennlinien, entlang derer die beschichtete Trägerfolienbahn 10 in mehrere Teilbahnen aufgetrennt wird. Der beschriebene erste Schritt des Verfahrens ist daher ein organisatorischer Schritt, der sich in den nachfolgend beschriebenen, die körperliche Ausgestaltung der beschichteten Trägerfolienbahn 10 verändernden, Herstellungsschritten widerspiegelt, aber nicht selbst einen solchen Herstellungsschritt bildet.

Figur 4 zeigt eine Ausführungsform einer Anordnung von Trennlinien 14 zum Auftrennen der beschichteten Trägerfolienbahn 10 in vier Teilbahnen 15a, 15b, 15c und 15d, von denen jede genau eine Reihe von wie in Figur 3 dargestellten Zellbereichen 11 enthält. Die Trennlinien sind der Wirkstoffdepot- bzw. Zellbereichsanordnung von Figur 3 entsprechend wellenförmig ausgebildet, wobei der Abstand Δu zwischen zwei aufeinanderfolgenden Kehrpunkten der Wellenlinien den Eingriff zweier an der jeweiligen Wellenlinie ineinander greifender Teilbahnen 15 angibt. Die den zweiten Schritt des oben bezeichneten Verfahrens bildende Auftrennung der Trägerfolienbahn 10 entlang der Trennlinien 14 erfolgt mit einer (nicht in den Figuren dargestellten) Schneid- oder Stanzeinrichtung. Das Auftrennen kann dabei diskontinuierlich mit z. B. einer Konturstanze oder kontinuierlich mit z. B. einer Konturschneidwalze vorgenommen werden.

Figur 5 illustriert die entlang der Trennlinien 14 in Teilbahnen 15a, 15b, 15c und 15d aufgetrennte Trägerfolienbahn 10, wobei die einzelnen Teilbahnen nach dem Auftrennen zudem seitlich soweit auseinandergeführt wurden, dass zueinander einander benachbarte Reihen nicht mehr ineinander eingreifen, d. h. Δu = 0, wobei der Abstand zwischen den Reihen gegebenenfalls auch größer gewählt werden kann. Damit ist sichergestellt, dass sich die einzelnen Teilbahnen bei dem im nachfolgenden Schritt vorgenommenen Verschieben einzelner Teilbahnen zum Ausgleich des Längsversatzes Δl bestenfalls punktuell berühren, jedoch nicht gegenseitig teilweise abdecken können. Alternativ zum seitlichen Versetzen der Teilbahnen kann bei Ausführungsformen zum Aufheben des Eingriffs auch jede zweite der Teilbahnen in eine andere Ebene überführt werden. Zum Eliminieren des Längsversatzes zwischen Wirkstoffdepots 12 bzw. Zellbereichen 11 benachbarter Reihen wird jede zweite der Teilbahnen relativ zu den anderen um eine dem Längsversatz Δl entsprechende Strecke versetzt bzw. verschoben. Beispielsweise können bei einer vierreihigen Anordnung wie in Figur 6 illustriert die Randteilbahn 15a und die zu dieser übernächste Teilbahn 15c relativ zu den anderen beiden Teilbahnen 15b und 15d um eine dem Längsversatz Δl entsprechende Strecke in Längsrichtung l der Trägerfolienbahn 10 verschoben werden. Die Verschiebung kann in einer Herstellungsanlage beispielsweise durch verschieden lange Laufstrecken der Teilbahnen realisiert werden.

Im dem Längsversatzausgleich nachfolgenden Schritt können die einzelnen Teilbahnen der wirkstoffhaltigen Beschichtung 2 auf eine neue Trägerfolie, vorzugsweise auf eine Schutzfolie umkaschiert werden. Jede der einer Teilbahn zugeordneten Schutzfolien kann dabei mehrteilig ausgeführt sein, worunter zu verstehen ist, dass sie aus mehreren Teilbahnen aufgebaut ist, von denen zumindest jeweils zwei so aneinander angrenzen oder sich überlappen, dass sie eine geschlossene Fläche bilden. Die Trennlinie zwischen den beiden Schutzfolienteilen bzw. deren Überlappungsbereich ist dabei vorzugsweise so angeordnet, dass er von den für die Ausbildung der Wirkstoffdepots 12 vorgesehenen Flächen überdeckt wird.

Im daran anschließenden Schritt werden die Wirkstoffdepots 12 in den wirkstoffhaltigen Beschichtungsteilbahnen vereinzelt. Dies erfolgt mittels Durchtrennen der wirkstoffhaltigen Beschichtung 2 entlang von in sich geschlossenen linienförmigen Geometrien, wobei jede dieser Geometrien jeweils in einem, einem Zellbereich 11 zugeordneten Gebiet der Beschichtung 2 angeordnet ist. Da die Wirkstoffdepots 12 bestimmte Konturen bzw. Formate aufweisen, wird dieser Schritt auch als Kontur- oder Formatstanzen bezeichnet.

Schließlich werden die Gitter 13 von den Beschichtungsteilbahnen in einem allgemein als Abgittern bezeichneten Vorgang abgezogen. Falls beim vorhergehenden Umkaschieren der Beschichtungsteilbahnen eine einteilige Schutzfolie verwendet wurde, wird diese nach dem Abgittern durch Längsschneiden in eine mehrteilige Schutzfolie überführt, wobei die Schutzfolie hierdurch zum einen in mehrere, jeweils eine Reihe von Wirkstoffdepots tragende, Teilbahnen aufgetrennt und zum anderen jede dieser Teilbahnen mit einem unter den Wirkstoffdepots 12 entlangführenden Trennschnitt oder einer Perforierung versehen werden kann. In einem nachfolgenden Bearbeitungsschritt werden die Schutzfolienbahnen in den Bereichen zwischen den Wirkstoffdepots in Querrichtung zum Erhalt von Einzelsystemen durchtrennt. Weitere fakultative Verarbeitungsschritte können gegebenenfalls ein Bedecken der Wirkstoffdepots 12 mit einer wirkstoffundurchlässigen Deck- bzw. Rückschichtfolie und ein Verpacken der Einzelsysteme umfassen.

Die Reihenfolge der oben beschriebenen Verfahrensschritte ist nicht bindend und kann zur Anpassung an Besonderheiten einer Anlage zum Herstellen transdermaler therapeutischer Systeme oder aus anderen Überlegungen heraus, wie beispielsweise Materialersparnis, Kontaminationsminimierung und dergleichen, geändert werden.

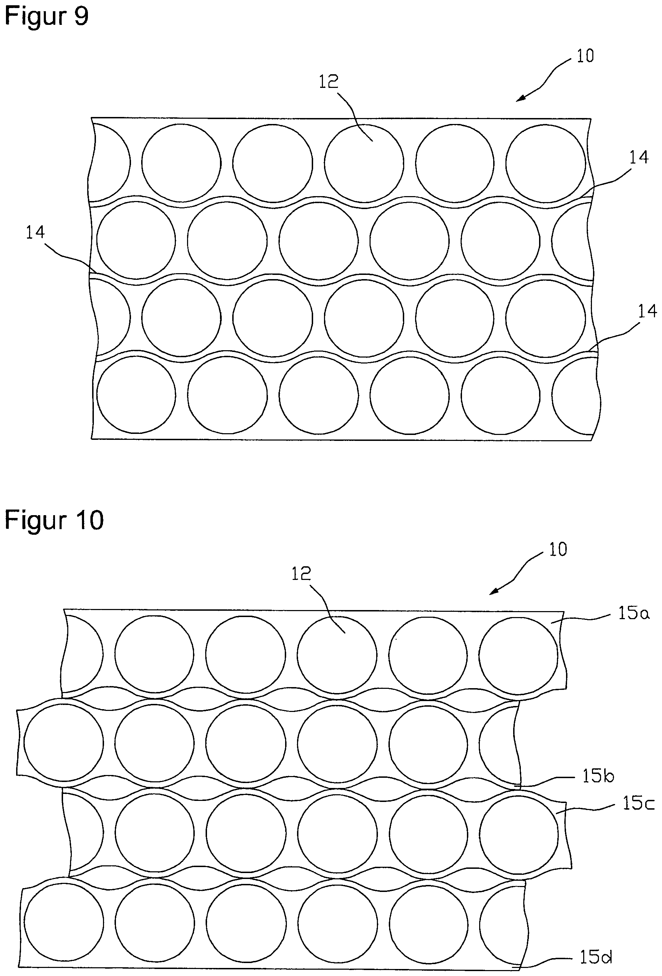

Beispielsweise kann das zuvor beschriebene Verfahren zur besseren Nutzung der wirkstoffhaltigen Beschichtung auch in einer Reihenfolge der Verfahrensschritte ausgeführt werden, bei der nach dem (zumindest gedanklich) vorgenommenen Schritt zum Untergliedern der Trägerfolie 10 in einzelne Zellbereiche 11 zunächst das Formatstanzen zur Vereinzelung der Wirkstoffdepots 12, wie es z. B. in Figur 8 veranschaulicht ist, vorgenommen wird. Im Anschluss daran kann das Gitter 13 von der Trägerfolie 1 abgenommen werden, woraufhin das in Figur 9 illustrierte Auftrennen der Trägerfolie 10 entlang der durch die Zellbereichsanordnung bestimmten Trennlinien 14 erfolgt. Nachfolgend werden die einzelnen Teilbahnen lateral (d. h. quer zur Längsrichtung l der Trägerfolienbahn 10) beabstandet und der Längsversatz zwischen einander unmittelbar benachbarten Teilbahnen ausgeglichen, so dass man z. B. eine Anordnung der formatgestanzten Teilbahnen 15a, 15b, 15c und 15d gemäß der schematischen Darstellung von Figur 10 erhält. Die gestanzten Beschichtungsteilbahnen werden nach dem Längsversatzausgleich auf eine Schutzfolie umkaschiert, bevor diese durch Längsschneiden oder Längsstanzen in wie oben beschriebene, eventuell mehrteilige Teilbahnen aufgetrennt wird. Schließlich werden auch bei dieser Verfahrensabfolge die Schutzfolienteilbahnen zur Vereinzelung der Systeme wie oben beschrieben quergeschnitten.

Bei einem weiteren beispielhaften Verfahrensablauf werden die Teilbahnen nach dem Auftrennen der Trägerfolienbahn wie beim oben erläuterten ersten Ausführungsbeispiel zunächst zur Aufhebung von Eingriff Δu und Längenversatz Δl relativ zueinander verschoben, bevor die hierdurch geschaffenen Beschichtungsteilbahnen jeweils auf eine gegebenenfalls mehrteilige Schutzfolienbahn übertragen werden. Anschließend werden die Wirkstoffdepots durch Konturstanzen der umkaschierten Beschichtungsteilbahnen ausgebildet, und die nicht als Wirkstoffdepot genutzten Teile der Beschichtungsteilbahnen abgegittert, bevor die Vereinzelung der Systeme durch Querschneiden der Schutzfolienbahnen erfolgt.

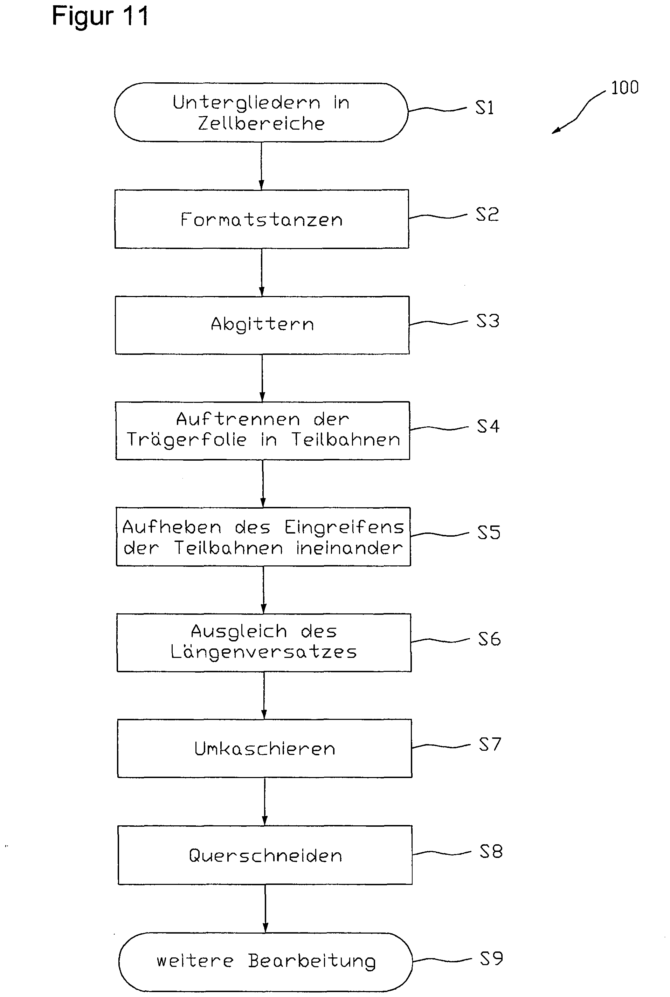

Eine übersichtliche Darstellung der einzelnen Schritte eines Verfahrens 100 zur besseren Nutzung der wirkstoffhaltigen Beschichtung 2 gemäß dem zweitgenannten Verfahrensablauf zeigt das Flussdiagramm der Figur 11. Das Verfahren 100 beginnt in Schritt S1 mit dem Untergliedern der Trägerfolie 10 in einzelne Zellbereiche 11, von denen jeder einem einzelnen Wirkstoffdepotformat 12 zugeordnet ist. Es sei noch einmal darauf verwiesen, dass dieser Schritt ausschließlich konzeptioneller Natur ist, und keine körperliche Veränderung der beschichteten Trägerfolie 10 beinhaltet bzw. beinhalten muss. In Schritt S2 erfolgt das Formatstanzen zur Ausbildung der einzelnen Wirkstoffdepots 12 in der Beschichtungslage 2 bzw. gegebenenfalls in der mit einer Deckfolie 3 versehenen Beschichtungslage 2. Im nachfolgenden Schritt S3 wird der nicht für Wirkstoffdepots 12 genutzte Teil 13 der eventuell mit einer Deckfolie 3 beschichteten wirkstoffhaltigen Beschichtungslage 2 abgegittert. Nach Schritt S3 erfolgt in Schritt S4 das Auftrennen der die Wirkstoffdepots 12 tragenden Trägerfolie 10 entlang der Trennlinien 14 in einzelne Teilbahnen, beispielsweise in Teilbahnen 15a, 15b, 15c und 15d. Im darauffolgenden Schritt S5 wird der Eingriff der Teilbahnen aufgehoben und anschließend oder gleichzeitig in Schritt S6 der Längsversatz zwischen den Teilbahnen ausgeglichen, wonach quer zur Längsrichtung der Teilbahnen nebeneinander angeordnete Zellbereiche 11 auf gleicher Höhe, d. h. ohne Längsversatz zueinander angeordnet sind. Die Aufhebung des Eingriffs Δu bzw. des Eingreifens der Teilbahnen ineinander kann durch Vergrößerung des Abstands zwischen den Teilbahnen quer zur Längsausdehnung der Teilbahnen als auch Überführen einer jeden zweiten Teilbahn in eine andere Ebene erfolgen. Im letzteren Fall werden die Wirkstoffdepots im späteren Verlauf (Schritt S7) auf separate Schutzfolienbahnen umkaschiert. Die Aufhebung des Längsversatzes zwischen den Wirkstoffdepots tragenden Teilbahnen kann durch Führen benachbarter Teilbahnen über unterschiedlich lange Teilstrecken realisiert werden. In Schritt S7 werden die Wirkstoffdepots 12 der einzelnen Teilbahnen auf eine oder mehrere Schutzfolienbahnen oder eine oder mehrere andere Trägerfolienbahnen umkaschiert. In einem später erfolgenden Schritt S8 werden die eventuell mehrteiligen Schutzfolien zur Vereinzelung der Systeme quergeschnitten und in Schritt S9 werden die Systeme der weiteren Bearbeitung zugeführt.

Das Verfahren 100 kann in der beschriebenen Abfolge von Verfahrensschritten als auch in einer wie oben beschrieben oder anders davon abweichenden sinnvollen Abfolge von Verfahrensschritten ausgeführt werden. So ist es beispielsweise möglich, in dem in Figur 11 gezeigten Verfahrensablauf die Schritte S7 und S8 auszulassen. Alternativ kann aus den Teilbahnen nach Schritt S6 direkt durch Querschneiden ohne den in Figur 11 gezeigten Zwischenschritt S7 das gewünschte Produkt zur weiteren Bearbeitung gewonnen werden. Dementsprechend können die Verfahrensschritte nach Schritt S6 vom Fachmann nach den jeweiligen Anforderungen und Zielsetzungen frei gewählt werden.

Das beschriebene Herstellungsverfahren ermöglicht eine verbesserte Nutzung einer auf einer Trägerfolie aufgebrachten wirkstoffhaltigen Beschichtung zur Herstellung transdermaler therapeutischer Systeme, wobei die zur Realisierung des Verfahrens erforderlichen Vorrichtungskomponenten in bestehende Anlagen zur Herstellung von transdermalen oder vergleichbaren Systemen für eine transdermale oder permukosale Wirkstoffverabreichung integriert werden können. Das beschriebene Herstellungsverfahren kann auch in einfacher Weise z. B. für die Herstellung von orodispersiven Tabletten (ODT) von zumeist runder Form angepasst werden.

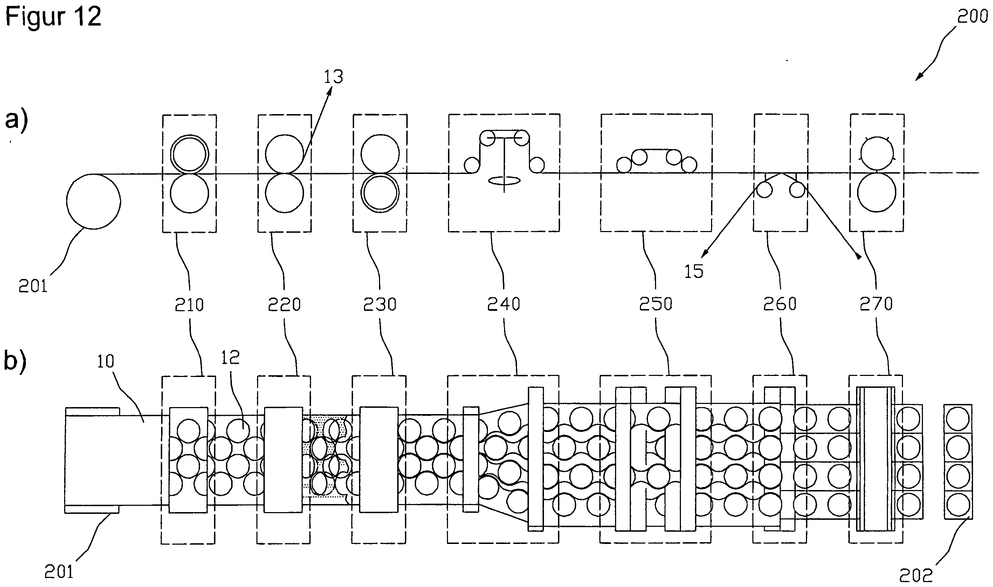

In den funktionsschematisierten Darstellungen von Figur 12 sind die für die Durchführung eines wie oben beschriebenen Verfahrens wesentlichen Einrichtungen einer Vorrichtung 200 der Offenbarung zur Herstellung transdermaler therapeutischer Systeme schematisch dargestellt.

Darstellung a) zeigt die Einrichtungen in einer Seitenansicht und Darstellung b) in einer Draufsicht. Die gezeigte Anordnung der Einrichtungen relativ zueinander ist in Bezug auf den in Figur 11 illustrierten Verfahrensablauf gewählt und kann bei entsprechend anders gewählten Verfahrensabläufen entsprechend variieren. Die nachfolgend beschriebenen Einrichtungen sind in der Figur zur besseren Erkennbarkeit jeweils mit einem gestrichelten Rahmen hervorgehoben.

Die Vorrichtung 200 der Offenbarung umfasst eine Konturierungseinrichtung 210 zum Vereinzeln von Wirkstoffdepots 12 in der gegebenenfalls mit einer Deckschicht 3 versehenen Beschichtungslage 2 mittels Format- bzw. Konturstanzen, eine Abgittereinrichtung 220 zum Entfernen des üblicherweise als Gitters bezeichneten, nicht als Wirkstoffdepot 12 genutzten Teils der gegebenenfalls mit einer Deckschicht 3 versehenen Beschichtungslage 2 von der Trägerfolienbahn 1 und eine Trenneinrichtung 230 zum längsweisen Auftrennen der Trägerfolienbahn 1 in zwei oder mehr Teilbahnen 15. Zum Aufheben eines Eingreifens Δu der Teilbahnen 15 ineinander kann die Versetzeinrichtung 240 wie veranschaulicht Schwenkrahmen umfassen, die die einzelnen Teilbahnen 15 seitlich, d. h. quer zu deren Längs- bzw. Transportrichtung, entsprechend weit auseinanderführt. Andere Offenbarungen einer Versetzeinrichtung 240 führen benachbarte Teilbahnen 15 über unterschiedliche Walzen, die so angeordnet sind, dass einander benachbarte Teilbahnen 15 auf verschiedene Ebenen geführt werden. In der Ausgleichseinrichtung 250 werden benachbarte Teilbahnen 15 über unterschiedlich lange Streckenabschnitte geführt, wobei die Unterschiede in den Streckenabschnitten im Wesentlichen dem ursprünglichen Längsversatz Δl der Wirkstoffdepots 12 benachbarter Teilbahnen 15 entsprechen. In der Umkaschiereinrichtung 260 werden die Wirkstoffdepots 12 auf eine wie oben beschrieben gegebenenfalls mehrteilige Schutzfolie umkaschiert. Insbesondere bei Verwendung einer Versetzeinrichtung 240, die die Teilbahnen ohne seitliches Auseinanderführen in verschiedene Ebenen überführt, ist die Umkaschiereinrichtung 260 zur Zuführung einer den Teilbahnen 15 entsprechenden Anzahl von gegebenenfalls mehrteiligen Schutzfolien an die in verschiedenen Ebenen geführten Teilbahnen ausgebildet. Zum Vereinzeln der Wirkstoffdepots 12 tragenden Schutzfolienbahn bzw. -bahnen in einzelne transdermale therapeutische oder andere Systeme 202 zur perkutanen Verabreichung von Wirkstoffen, weist die Vorrichtung 200 der Offenbarung schließlich eine Querschneideeinrichtung 270 auf, die zum Durchtrennen der gegebenenfalls zuvor mit einer (nicht dargestellten) Längsschneideeinrichtung in mehrere Bahnen aufgeteilten Schutzfolie quer zur Längsrichtung in den Bereichen zwischen den Wirkstoffdepots 12 ausgebildet ist. Den beschriebenen Einrichtungen wird die beschichtete Trägerfolienbahn 10 vorzugsweise über eine Wickelrolle 201 zugeführt.

Es wird offenbart, dass die Konturierungseinrichtung 210 eine Stanzvorrichtung mit Stanzklingen ist oder umfasst, welche in einigen Fällen fest (d.h. unveränderbar) mit der Stanzvorrichtung verbunden sind. Die Stanzklingen der Stanzvorrichtung sind so angebracht, dass beim Formatstanzen im Schritt S2 eine wie zuvor beschriebene Anordnung der Wirkstoffdepots 12 mit optimierter Flächennutzung resultiert. So können die Stanzklingen insbesondere so angebracht sein, dass die in Figur 8 gezeigte Anordnung resultiert. Die in Figur 8 gezeigte Anordnung von Wirkstoffdepots 12 weist einen vorgegebenen Reihenabstand Δs wie auch einen vorgegebenen Längsversatz Δl auf, der jeweils größer als Null ist. Der Reihenabstand Δs und der Längsversatz Δl werden so gewählt, dass die Nutzung der wirkstoffhaltigen Beschichtung gegenüber der in Figur 2 gezeigten matrixförmigen Anordnung verbessert wird. Die Verbesserung erfolgt also durch Verminderung des Anteils des nicht von den Wirkstoffdepots genutzten Bereichs der wirkstoffhaltigen Beschichtung, d.h. durch Reduktion des Gitterverlusts.

Es wird offenbart, dass die Konturierungseinrichtung 210 eine Stanzwalze mit fest angebrachten Stanzklingen ist oder umfasst, wobei die Stanzklingen so angeordnet sind, dass der Gitterverlust reduziert wird. Die Stanzklingen werden typischerweise so angeordnet, dass die durch das Formatstanzen in Schritt (S2) ausgebildete Anordnung von Wirkstoffdepots (12) einen vorgegebenen Reihenabstand Δs und einen vorgegebenen Längsversatz Δl aufweist, der jeweils größer als Null ist und der jeweils so gewählt wird, dass der Gitterverlust reduziert wird.

Die Stanzklingen der Stanzvorrichtung können so angeordnet sein, dass bei Verwendung der Stanzvorrichtung beim Formatstanzen in Schritt (S2) in Reihen angeordnete, vorzugsweise runde oder ovale Wirkstoffdepots (12) ausgebildet werden, wobei die Reihen so ineinander eingreifen, dass der zwischen zwei benachbarten Wirkstoffdepots 12 einer Reihe verfügbare Raum von einem Wirkstoffdepot 12 einer zu dieser Reihe unmittelbar benachbart angeordneten Reihe mitgenutzt wird.

## Patentansprüche

1. Verfahren zur Herstellung von Systemen zur transdermalen oder permukosalen Verabreichung von Wirkstoffen, wobei das Verfahren folgende Schritte umfasst:
- Bereitstellen einer beschichteten Trägerfolienbahn (10), umfassend eine Trägerfolienbahn (1) mit einer darauf haftklebenden wirkstoffhaltigen Beschichtung (2), wobei auf der beschichteten Trägerfolienbahn (10) Wirkstoffdepotflächen (12) derart definiert sind, dass die Wirkstoffdepotflächen (12) in Bahnrichtung (l) der beschichteten Trägerfolienbahn (10) in zwei oder mehr Reihen derart angeordnet sind, dass sich die Reihen in Bahnrichtung (1) nicht mittels einer geraden Linie voneinander trennen lassen, ohne dabei Wirkstoffdepotflächen (12) zu schneiden, und dass der Anteil der Oberfläche der beschichteten Trägerfolienbahn (10), der nicht als Wirkstoffdepotfläche (12) genutzt wird, unter Nichtberücksichtigung der Randzonen der beschichteten Trägerfolienbahn (10), weniger als 39 % der Gesamtfläche der beschichteten Trägerfolienbahn (10) beträgt; und
- Durchtrennen (S4) der beschichteten Trägerfolienbahn (10) in Bahnrichtung (1) in zwei oder mehr Teilbahnen (15a, 15b, 15c, 15d) derart, dass jede Teilbahn eine Reihe von Wirkstoffdepotflächen (12) enthält, wobei beim Durchtrennen keine der Wirkstoffdepotflächen (12) geschnitten wird.

2. Verfahren gemäß Anspruch 1, wobei das Verfahren mindestens einen der folgenden weiteren Schritte umfasst:
- Verändern (S5) der Lage der Teilbahnen (15a, 15b, 15c, 15d) relativ zueinander so, dass keine der Teilbahnen seitlich in eine der anderen Teilbahnen eingreift;
- Verschieben (S6) der Teilbahnen (15a, 15b, 15c, 15d) relativ zueinander in Bahnrichtung (1) so, dass die parallel liegenden Teilbahnen in einer Richtung 90° zur Bahnrichtung (1) in geraden Linien so durchtrennt werden können, dass Einzelabschnitte erhalten werden, die jeweils nur eine Wirkstoffdepotfläche (12) enthalten, und wobei beim Durchtrennen keine der Wirkstoffdepotflächen (12) geschnitten wird.

3. Verfahren gemäß einem der Ansprüche 1 bis 2, wobei die Wirkstoffdepotflächen (12) so angeordnet sind, dass die Anordnung keine vierzählige Drehachse aufweist, vorzugsweise so, dass die Anordnung die Symmetrieelemente der Gruppe p6m aufweist.

4. Verfahren gemäß einem der Ansprüche 1 bis 3, wobei die Wirkstoffdepotflächen (12) durch Formstanzen (S2) der beschichteten Trägerfolienbahn (10) definiert werden, und das Verfahren optional folgenden weiteren Schritt umfasst:
- Entfernen (S3) (Abgittern) der nicht als Wirkstoffdepotflächen (12) definierten Bereiche der beschichteten Trägerfolienbahn (10).

5. Verfahren gemäß einem der Ansprüche 1 bis 4, worin die Wirkstoffdepots bzw. Wirkstoffdepotflächen kreisförmig oder ellipsenförmig ausgebildet sind und/oder worin die Wirkstoffdepots bzw. Wirkstoffdepotflächen alle gleich groß sind.

## Claims

1. A method for producing systems for the transdermal or permucosal administration of active substances, wherein the method comprises the following steps:
- providing a coated carrier film web (10) comprising a carrier film web (1) with an active substance-containing coating (2) adherent thereon, wherein active substance depot areas (12) are defined on the coated carrier film web (10) such that the active substance depot areas (12) are arranged in the web direction (l) of the coated carrier film web (10) in two or more rows such that the rows cannot be separated by means of a straight line without thereby cutting the active substance depot areas (12), and such that the proportion of the surface of the coated carrier film web (10) that is not used as active substance depot area (12) by ignoring the rim zones of the coated carrier film web (10) is less than 39 % of the total area of the coated carrier film web (10); and
- severing (S4) the coated carrier film web (10) in the web direction (l) into two or more sub-webs (15a, 15b, 15c, 15d) such that each sub-web contains a row of active substance depot areas (12), wherein upon severing none of the active substance depot areas (12) is cut.

2. The method according to claim 1, wherein the method comprises at least one of the following further steps:
- changing (S5) the location of the sub-webs (15a, 15b, 15c, 15d) relative to each other such that none of the sub-webs laterally engages one of the other sub-webs.
- displacement (S6) of the sub-webs (15a, 15b, 15c, 15d) relative to each other in the web direction (l) such that the parallel lying sub-webs in a direction 90° to the web direction (l) can be severed in straight lines such that individual portions are obtained each containing only one active substance depot area (12), and wherein upon severing none of the active substance depot areas (12) is cut.

3. The method according to anyone of claims 1 to 2, wherein the active substance depot areas (12) are arranged such that the arrangement has no tetrad rotational axis, preferably such that the arrangement has the group p6m symmetry elements.

4. The method according to anyone of claims 1 to 3, wherein the active substance depot areas (12) are defined by pressure forming (S2) of the coated carrier film web (10) and wherein the method optionally comprises the following further step:
- removing (S3) (weeding) the regions of the coated carrier film web (10) that are not defined as active substance depot areas (12).

5. The method according to anyone of claims 1 to 4, wherein the active substance depots or active substance depot areas, respectively, are circular or elliptical and/or wherein the active substance depots or active substance depot areas, respectively, are all equal in size.

## Revendications

1. Procédé servant à produire des systèmes servant à l'administration transdermique ou per-muqueuse de substances actives, le procédé comprenant les étapes suivantes consistant à :
- fournir une bande de film de support (10) revêtue, comprenant une bande de film de support (1) pourvue d'un revêtement (2) contenant une substance active et adhérant à la dite bande de film de support, des surfaces de dépôt de substance active (12) étant définies sur la bande de film de support (10) revêtue de telle manière que les surfaces de dépôt de substance active (12) sont disposées dans le sens de bande (1) de la bande de film de support (10) revêtue en deux rangées ou plus de telle manière que les rangées ne peuvent être séparées les unes des autres dans le sens de bande (1) au moyen d'une ligne rectiligne sans couper ce faisant des surfaces de dépôt de substance active (12), et en ce que la proportion de la surface de la bande de film de support (10) revêtue, qui n'est pas utilisée en tant que surface de dépôt de substance active (12), est égale, sans tenir compter des zones en bordure de la bande de film de support (10) revêtue, à moins de 39 % de la surface totale de la bande de film de support (10) revêtue, et
- séparer (S4) la bande de film de support (10) revêtue dans le sens de la bande (1) en deux bandes partielles ou plus (15a, 15b, 15c, 15d) de telle manière que chaque bande partielle contient une rangée de surfaces de dépôt de substance active (12), aucune des surfaces de dépôt de substance (12) active n'étant coupée lors de la séparation.

2. Procédé selon la revendication 1, le procédé comprenant au moins une des autres étapes qui suivent consistant à :
- modifier (S5) la position des bandes partielles (15a, 15b, 15c, 15d) les unes par rapport aux autres de sorte qu'aucune des bandes partielles ne vienne en prise sur le côté avec une des autres bandes partielles ;
- déplacer par coulissement (S6) les bandes partielles (15a, 15b, 15c, 15d) les unes par rapport aux autres dans le sens de la bande (1) de sorte que les bandes partielles situées de manière parallèle les unes par rapport aux autres puissent être séparées, dans une direction à 90° par rapport au sens de la bande (1), en des lignes rectilignes de sorte que des sections individuelles sont obtenues, lesquelles contiennent respectivement uniquement une surface de dépôt de substance active (12), et aucune des surfaces de dépôt de substance active (12) n'étant coupée lors de la séparation.

3. Procédé selon l'une quelconque des revendications 1 à 2, les surfaces de dépôt de substance active (12) étant disposées de telle sorte que l'agencement ne présente aucun axe de rotation à quatre tours, de préférence de telle sorte que l'agencement présente les éléments de symétrie du groupe p6m.

4. Procédé selon l'une quelconque des revendications 1 à 3, les surfaces de dépôt de substance active (12) étant définies par un nervurage à la presse (S2) de la bande de film de support (10) revêtue, et le procédé comprenant en option les étapes qui suivent consistant à :
- éloigner (S3) (séparer par une grille) les zones non définies en tant que surfaces de dépôt de substance active (12) de la bande de film de support (10) revêtue.

5. Procédé selon l'une quelconque des revendications 1 à 4, dans lequel les dépôts de substance active ou les surfaces de dépôt de substance active sont réalisés de manière à présenter une forme circulaire ou une forme elliptique, et/ou dans lequel les dépôts de substance active ou les surfaces de dépôt de substance active présentent tous une dimension identique.
